# EUROPEAN PATENT APPLICATION

(11) **EP 4 316 623 A1**
(43) Date of publication of application: **07.02.2024**
(21) Application number: 22188651.8
(22) Date of filing: 04.08.2022
(51) Int. Cl.: B01D 15/16, B01D 15/12, B01D 15/30, B01D 15/34, B01J 20/28, C07K 1/16, C12N 7/02

(54) **CHROMATOGRAPHY METHOD FOR NANOPARTICLE PURIFICATION AND/OR SEPARATION**

(71) Applicant: Max-Planck-Gesellschaft zur Förderung der Wissenschaften e.V., 80539 München (DE)
(72) Inventor: MARICHAL-GALLARDO, Pavel Alejandro, 39114 Magdeburg (DE)
(74) Representative: Kuttenkeuler, David

(57) **Abstract**

The present invention pertains to a chromatography method for the purification and/or separation of target nanoparticles. Said method relies on a hydrophilic stationary phase that comprises rigid beads with throughpores, making these beads compatible with highly viscous solvents. Furthermore, the invention pertains to compositions comprising purified and/or separated nanoparticles and their use in medicine and/or manufacturing of medicaments or vaccines. Additionally, the invention pertains to a method for the depletion of nanoparticles from a target composition, wherein said hydrophilic stationary phase that comprises rigid beads with throughpores is used, and a nanoparticle-depleted composition obtainable by said method.

## Description

### FIELD OF THE INVENTION

The present invention pertains to a chromatography method for the purification and/or separation of target nanoparticles. Said method relies on a hydrophilic stationary phase that comprises rigid beads with throughpores, making these beads compatible with highly viscous solvents. Furthermore, the invention pertains to compositions comprising purified and/or separated nanoparticles and their use in medicine and/or manufacturing of medicaments or vaccines. Additionally, the invention pertains to a method for the depletion of nanoparticles from a target composition, wherein said hydrophilic stationary phase that comprises rigid beads with throughpores is used, and a nanoparticle-depleted composition obtainable by said method.

### DESCRIPTION

In chromatography methods that rely on traditional porous beads packed in a column, there are two main modes of mass transport. The inter-bead space is dominated by convective flow, which is a movement induced by external force, such as by a pump, and is depending on the flow rate in the column. Mass transport in the pores of the beads is governed by diffusion flow, a random thermal movement leading to an equilibrium between areas of higher solute concentration and lower solute concentration.

Steric exclusion chromatography (SXC) is a powerful method for the concentration and purification of nanoparticles such as virus particles used in vaccines and gene therapies. In this chromatography method, nanoparticles are dispersed in a solvent together with a second solute, typically a non-ionic polymer such as a PEG. The steric exclusion of the nanoparticles and the second solute leads to the nanoparticles being surrounded by a deficiency zone. In this deficiency zone, the second solute has a lower concentration than in the bulk solvent, which leads to an unfavourable increase in free energy. In the method, a stationary phase is provided, to which the nanoparticles can attach in order to minimize the free energy.

As a chromatography method, SXC utilizes highly porous structures as stationary phases, namely membranes and monoliths, whose use has been reported in literature as recently as the last decade. Due to mass transport phenomena relating to SXC and certain operational characteristics of the method, SXC has been deemed impractical if not impossible to execute when attempted with legacy chromatography supports such as beads in packed columns. For example, a 10% solution of PEG 6000 at 25°C is already more than 4 times more viscous, a 20% PEG 6000 solution is more than 11 times more viscous than water. As solvent viscosity reduces solute diffusivity in direct proportion, it severely handicaps diffusive mass transfer in chromatography columns packed with porous particles.

Furthermore, molecular shear forces in the inter-particle (void) space also increase proportionally with viscosity, and operating pressures become prohibitive. Chromatography beads are typically made of soft materials like agarose and were originally designed for the purification of proteins, not viruses or other much larger nanoparticles. Beads have several limitations for purification of viruses: Amongst others, the soft structure of traditional beads limits their use under pressure. Attempts to perform SXC on porous particles were practical failures typically accompanied by clogging and compacting of stationary phases and pressure surges. In the viscous buffers of SXC, this results in a slow speed and low productivity. These limitations are circumvented by membranes and monoliths, making them the best choice for purification of virus particles.

Despite these apparent disadvantages, the inventors demonstrate that SXC can be successfully performed with beads in packed columns. These beads, known as perfusion beads, are comprised of a rigid, non-compressible material with throughpores that transect the entire particle and may vary in their hydrophilic surface chemistries. The throughpores allow for convective mass transport into the pores of a bead, which increases the accessible bead surface to solute species and facilitates diffusive mass transport.

WO2017/076553 pertains to a method for the purification of virus compositions as well as biological macromolecular compounds in a sample comprising mixing the sample with osmolytes, like a non-ionic organic polymer, and contacting the mixed sample with a hydrophilic membrane, optionally washing the membrane, and eluting the virus preparations or biological macromolecular components from the membrane with an eluting solution containing reduced amounts or no osmolytes.

WO2018/041389 relates to a method for the purification of viruses or virus-like particles using a crosslinked cellulose hydrate membrane in the presence of a polyalkylene glycol, and to a kit for the purification of viruses or virus-like particles and the use thereof.

WO2012/169970 shows a method for purification of a hydrated target species of biological origin in a sample comprising the steps of contacting the sample with a hydrated surface of an undissolved material and contacting the sample with a constraining agent in an amount sufficient to cause at least a fraction of the target species to be retained at the hydrated surface of the undissolved material.

Using perfusion beads, the limitations of traditional beads that before made SXC practically impossible are overcome. SXC can be performed at high speeds (>2000 cm/h compared to traditional beads at <200-300 cm/h) and diffusion-based mass transport limitations are greatly reduced thanks to a semi-convective flow character and the rigidity of the perfusion beads. Results so far evidence the successful capture and purification of different virus products utilizing varying packed resins of different bead sizes, surface chemistries, and pore sizes.

Moreover, the invention offers advantages over the state-of-the-art supports (i.e., membranes and monoliths) used in the past for SXC, including a higher resolution (meaning more separation power) and a wider choice of surface chemistries over the typical stationary phases, greatly expanding for product selectivity and purification potential. Furthermore, due to their bead-nature they offer the advantage of being highly adaptable: they can be stored and/or transported in bulk and later portioned and formed to a stationary phase that fits a desired column.

Thus, it is an objection of the invention to provide a new and improved chromatography method for the purification and/or separation of target nanoparticles (e.g. virus particles) by using hydrophilic and rigid beads with throughpores as stationary phase, preferably packed in a chromatography column, and a mobile phase with a non-ionic dissolved polymer. The target nanoparticles are captured in the presence of the non-ionic dissolved polymer and can be recovered by using a solvent with a reduced amount of non-ionic dissolved polymer in the mobile phase. Similarly, said finding can be used in a method for the depletion of nanoparticles from a target composition.

### BRIEF DESCRIPTION OF THE INVENTION

Generally, and by way of brief description, the main aspects of the present invention can be described as follows:
In **the first aspect,** the invention pertains to a chromatography method for the purification and/or separation of target nanoparticles comprising the steps of:
(i) loading the target nanoparticles onto a hydrophilic stationary phase that comprises rigid beads with throughpores in the presence of a hydrophilic mobile phase that comprises a non-ionic dissolved polymer, thus attaching the target nanoparticles to the hydrophilic stationary phase;
(ii) optionally, washing the hydrophilic stationary phase with a hydrophilic washing phase that preferably also comprises a non-ionic dissolved polymer;
(iii) eluting the target nanoparticles from the hydrophilic stationary phase with an eluent;
and, preferably, wherein the hydrophilic stationary phase is packed in a chromatography device.

In **a second aspect,** the invention pertains to a composition comprising purified and/or separated nanoparticles, wherein the purified and/or separated nanoparticles are the target nanoparticles obtainable by the chromatography method according to the other aspects of the invention.

In **a third aspect,** the invention pertains to a composition comprising purified and/or separated nanoparticles for use in medicine, wherein the composition comprising purified and/or separated nanoparticles is a composition according to the second aspect of the invention.

In **a fourth aspect,** the invention pertains to a composition comprising purified and/or separated nanoparticles for use in the manufacturing of a medicament or a vaccine, wherein the composition comprising purified and/or separated nanoparticles is a composition according to the second aspect of the invention.

In a **fifth aspect,** the invention pertains to a method for the depletion of nanoparticles from a liquid target composition, comprising the steps:
(u) dissolving a non-ionic polymer according to any previous aspect of the invention in the liquid target composition in a concentration according to any previous aspect of the invention;
(v) bringing into contact the liquid target composition containing the non-ionic dissolved polymer with a hydrophilic stationary phase according to any previous aspect of the invention, thus allowing the nanoparticles to attach to the hydrophilic stationary phase and creating a nanoparticle-depleted target composition;
(w) eluting the nanoparticle-depleted target composition,
wherein the hydrophilic stationary phase is packed in a chromatography device and the nanoparticles are the target nanoparticles according to any previous aspect of the invention.

**In a sixth aspect,** the invention pertains to a nanoparticle-depleted composition, obtainable by the method for the depletion of nanoparticles according to the fifth aspect of the invention.

### DETAILED DESCRIPTION OF THE INVENTION

In the following, the elements of the invention will be described. These elements are listed with specific embodiments, however, it should be understood that they maybe combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

**In the first aspect,** the invention pertains to a chromatography method for the purification and/or separation of target nanoparticles comprising the steps of:
(i) loading the target nanoparticles onto a hydrophilic stationary phase that comprises rigid beads with throughpores in the presence of a hydrophilic mobile phase that comprises a non-ionic dissolved polymer, thus attaching the target nanoparticles to the hydrophilic stationary phase;
(ii) optionally, washing the hydrophilic stationary phase with a hydrophilic washing phase that preferably also comprises a non-ionic dissolved polymer;
(iii) eluting the target nanoparticles from the hydrophilic stationary phase with an eluent;
and, preferably, wherein the hydrophilic stationary phase is packed in a chromatography device.

The method of the invention thereby results in an eluate comprising the target nanoparticles in separated and/or purified form.

In one alternative of the first aspect, the invention further pertains to a method for purifying target nanoparticles from a solution, comprising the following steps:
x) providing a mixture M composed of a first solution and a second solution, the first solution containing the target nanoparticles to be purified and, optionally impurities, and the second solution containing a non-ionic dissolved polymer, such as a polyalkylene glycol having two or three carbon atoms in the repeating unit,
y) loading a chromatography support that is a hydrophilic stationary phase with the mixture M from step x), with the target nanoparticles and thereby attaching and retaining the target nanoparticles to the external and internal surfaces of the hydrophilic stationary phase;
y') optionally washing the hydrophilic stationary phase with a hydrophilic washing phase;
z) eluting the target nanoparticles retained in the hydrophilic stationary phase in an eluate using an eluent which contains no non-ionic dissolved polymer, or no polyalkylene glycol having two or three carbon atoms in the repeating unit or which contains at least 20% (preferably at least 50%) less non-ionic dissolved polymer or polyalkylene glycol having two or three carbon atoms in the repeating unit than the mixture M from step x), with the result that the target nanoparticles are obtained in purified form in the eluate;
characterized in that
the chromatography support is composed of rigid beads with throughpores.

The numbering of the steps (i) to (iii) indicates that these steps are conducted in a successive order.

In the context of the invention, the stationary phase refers to a porous phase that comprises a plurality of rigid beads with throughpores. Therefore, the stationary phase comprises throughpores. A "throughpore" in the context of the invention refers to a channel that transects the entire bead. However, this does not mean that a throughpore of the stationary phase needs to transect the entire stationary phase.

When conducting step (i) or preferably step y), the target nanoparticles that are to be purified and/or separated are attached to the hydrophilic stationary phase. The concept, on which this attachment relies on, is known as steric exclusion chromatography (SXC). Without wishing to be bound by theory, this chromatography functions as follows: In a thermodynamic system that comprises a solvent and multiple types solute species, multiple types of solute species that are not able to interpenetrate sterically exclude each other. A first solute species in this thermodynamic system is surrounded by a deficiency zone, in which a second solute species is present in a lower concentration compared to the bulk concentration of the second solute species in the solvent. This discontinuity between solute-rich zones and solute-poor zones creates an increase in free energy. The thermodynamic system can minimize the free energy by the reduction of interfaces between the solute-rich and solute-poor zones. This is achieved by providing a surface that solute species is able to attach to.

In preferred embodiments of the invention, the chromatography method is a steric exclusion chromatography (SXC). The present application, relies on the above-described thermodynamic system. It comprises a first solute species referred to as "target nanoparticles" and a second solute species referred to as "non-ionic dissolved polymer" and a surface referred to as "hydrophilic stationary phase". The first solute species "target nanoparticles" can attach to the hydrophilic stationary phase, therefore minimizing the free energy of the system. As a result of conducting step (i) or preferably step y), the target nanoparticles are attached to the hydrophilic stationary phase.

Preferably, the chromatography method of the invention is conducted in a chromatography system. In such a chromatography system, the hydrophilic stationary phase is fed with the target particles and the hydrophilic mobile phase that comprises the non-ionic dissolved polymer. The chromatography system comprises a module that contains the hydrophilic stationary phase, preferably wherein the module containing the hydrophilic stationary phase comprises at least on inlet and at least one outlet. Preferably, in the chromatography system, the hydrophilic mobile phase and the target nanoparticles are stored in separate reservoirs.

In the context of the invention, the hydrophilic stationary phase is preferably packed in a chromatography column. In the context of the present invention, the terms "column" and "chromatography column" are used interchangeably". Preferably, the chromatography method of the present invention is a column chromatography method.

The chromatography method of the present invention relies on flow of solvents such as the eluent, the hydrophilic mobile phase and the hydrophilic washing phase through the hydrophilic stationary phase. In the context of the invention, this flow defines a flow direction. Therefore, the terms "before" or "after" may refer in a spatial context to the flow direction and/or may refer to points in time.

Rigidity in the context of the invention is beneficial, since it increases the pressure and linear flow velocity that a material, bead, and/or stationary phase can be exposed to. This is especially advantageous in the methods of the present invention and in SXC in general, where the solvents (such as the hydrophilic mobile phase) are typically highly viscous as they contain non-ionic dissolved polymers.

The hydrophilic stationary phase of the invention comprises rigid beads with throughpores. When referring to the rigid beads, "rigidity" preferably refers to one or more of the following:
- the rigid beads with throughpores, when packed in a chromatography column with a chromatography column height of 30 cm and using a linear flow velocity of 350 cm/h, exhibit a volume change of less than 50%, preferably less than 40%, preferably less than 30%, preferably less than 20%, preferably less than 10%, most preferably less than 5% compared to their fully hydrated volume at standard pressure;
- the rigid beads with throughpores, when packed in a chromatography column with a chromatography column height of 30 cm and using a linear flow velocity of 350 cm/h, exhibit a change in their porosity of less than 50%, preferably less than 40%, preferably less than 30%, preferably less than 20%, preferably less than 10%, most preferably less than 5% compared to their porosity in a fully hydrated state at standard pressure;
- the rigid beads withstand a pressure of at least 10 bar, preferably at least 60 bar, preferably at least 100 bar, preferably at least 200 bar, most preferably at least 300 bar.

In this context, "withstanding" may refer to a volume change of less than 50%, preferably less than 40%, preferably less than 30%, preferably less than 20%, preferably less than 10%, most preferably less than 5% compared to the fully hydrated volume of the of the rigid beads with throughpores at standard pressure. In this context, "withstanding" may refer to a change in porosity of less than 50%, preferably less than 40%, preferably less than 30%, preferably less than 20%, preferably less than 10% , most preferably less than 5% of the rigid beads with throughpores compared to the porosity of the rigid beads with throughpores in a fully hydrated state at standard pressure.

Preferably, the hydrophilic stationary phase is a rigid hydrophilic stationary phase. When referring to the stationary phase, "rigidity" preferably refers to one or more of the following:
- the hydrophilic stationary phase, when packed in a chromatography column with a chromatography column height of 30 cm and using a linear flow velocity of 350 cm/h, exhibits a volume change of less than 50%, preferably less than 40%, preferably less than 30%, preferably less than 20%, preferably less than 10%, most preferably less than 5% compared to its fully hydrated volume at standard pressure;
- the hydrophilic stationary phase, when packed in a chromatography column with a chromatography column height of 30 cm and using a linear flow velocity of 350 cm/h, exhibits a change in its porosity of less than 50%, preferably less than 40%, preferably less than 30%, preferably less than 20%, preferably less than 10%, most preferably less than 5% compared to its porosity in a fully hydrated state at standard pressure;
- the hydrophilic stationary phase withstands a pressure of at least 10 bar, preferably at least 60 bar, preferably at least 100 bar, preferably at least 200 bar, most preferably at least 300 bar.

In this context, "withstanding" may refer to a volume change of less than 50%, preferably less than 40%, preferably less than 30%, preferably less than 20%, preferably less than 10%, most preferably less than 5% compared to the fully hydrated volume of the of the hydrophilic stationary phase at standard pressure. In this context, "withstanding" may refer to a change in porosity of less than 50%, preferably less than 40%, preferably less than 30%, preferably less than 20%, preferably less than 10%, most preferably less than 5% of the hydrophilic stationary phase compared to the porosity of the hydrophilic stationary phase in a fully hydrated state at standard pressure.

The above embodiments correlate the volume change or the porosity change of the hydrophilic stationary phase or the rigid beads with throughpores, when packed in a chromatography column with a chromatography column height of 30 cm and using a linear flow velocity of 350 cm/h, with the respective features of the hydrophilic stationary phase or the rigid beads with throughpores, when fully hydrated at standard pressure. Said embodiments are meant to disclose general thresholds to the features of the rigid beads with throughpores or the hydrophilic stationary phase. They do not limit the chromatography method to be operated using a chromatography column, a flow velocity of 350 cm/h and a chromatography column height of 30 cm and they do not limit the chromatography method to be conducted at a fully hydrated state of the rigid beads with throughpores or the hydrophilic stationary phase at standard pressure.

Preferably, the rigid beads with throughpores are non-gel rigid-beads with throughpores. When referring to the non-gel rigid beads with throughpores, "non-gel" or "not a gel" preferably refers to a property according to which the rigid beads with throughpores, when fully hydrated, comprise a volume increase of less than 50%, preferably less than 40%, most preferably less than 30%, when compared to the rigid beads with throughpores in a dried state.

Preferably, the hydrophilic stationary phase is a non-gel hydrophilic stationary phase. In this context, "non-gel" or "not a gel" refers to a property according to which the hydrophilic stationary phase, when fully hydrated, comprises a volume increase of less than 50%, preferably less than 40%, most preferably less than 30%, when compared to the hydrophilic stationary phase in a dried state.

In particular, a non-gel hydrophilic stationary phase and/or a rigid bead with throughpores may be advantageous as it features a constant pore size distribution, regardless of the hydration state of the column. Moreover, a column made from such a hydrophilic stationary phase and/or a rigid bead with throughpores can be prepared faster.

Preferably, the expression "non-gel" does not exclude that the described object is a substantially dilute cross-linked system, which exhibits no flow when in the steady-state although the liquid phase may still diffuse through this system. Furthermore, the expression "non-gel" does not exclude that the described object is a nonfluid colloidal network or polymer network that is expanded throughout its whole volume by a fluid.

The above embodiments correlate the volume of the hydrophilic stationary phase or the rigid beads with throughpores, when fully hydrated to the volume of hydrophilic stationary phase or the rigid beads with throughpores in a dried state. Said embodiments do not limit the chromatography method to be operated at a "fully hydrated" state. Furthermore, they do not limit the chromatography method to be operated at a dried state of the rigid beads with throughpores or of the hydrophilic stationary phase.

Preferably, the hydrophilic stationary phase is packed in a chromatography column. In preferred embodiments of the invention, the maximum height of the chromatography column is limited by a volume change of the hydrophilic stationary phase and/or the rigid beads with throughpores when the chromatography column is operated. Preferably, when the chromatography column is operated, the volume change of the hydrophilic stationary phase and/or rigid beads with throughpores, is less than 50%, preferably less than 40%, preferably less than 30%, preferably less than 20%, preferably less than 10%, most preferably less than 5% when compared to their fully hydrated volume at standard pressure.

In preferred embodiments of the invention, the maximum height of the chromatography column is limited by a change in porosity of the hydrophilic stationary phase and/or the rigid beads with throughpores when the chromatography column is operated. Preferably, when the chromatography column is operated, the change in porosity of the hydrophilic stationary phase and/or rigid beads with throughpores, is less than 50%, preferably less than 40%, preferably less than 30%, preferably less than 20%, preferably less than 10%, most preferably less than 5% compared to the porosity of the hydrophilic stationary phase and/or the rigid beads with throughpores in a fully hydrated state at standard pressure.

Preferably, the height of the chromatography column is selected from to 5 to 50 cm, more preferably 5 cm to 40 cm, most preferably 10 cm to 30 cm. Preferably, the height of the chromatography column is 10 cm, 20 cm or 30 cm. Most preferably, the height of the chromatography column is 30 cm.

In preferred embodiments of the invention, hydrophilic mobile phase features a linear flow velocity of at least 10 cm/h, at least 30 cm/h, at least 50 cm/h, at least 100 cm/h, at least 200 cm/h, at least 350 cm/h, at least 500 cm/h, at least 1000 cm/h, at least 1500 cm/h most preferably at least 2000 cm/h. In preferred embodiments of the invention, hydrophilic mobile phase features a linear flow velocity of at least 350 cm/h.

In preferred embodiments, the maximum linear flow velocity is limited by a volume change of the hydrophilic stationary phase and/or the rigid beads with throughpores when operated at said linear flow velocity. Preferably, when operated at said linear flow velocity, the volume change of the hydrophilic stationary phase and/or the rigid beads with throughpores, is less than 50%, preferably less than 40%, preferably less than 30%, preferably less than 20%, preferably less than 10%, most preferably less than 5% compared to their fully hydrated volume at standard pressure.

In preferred embodiments, the maximum linear flow velocity is limited by a change in porosity of the hydrophilic stationary phase and/or the rigid beads with throughpores when operated at said linear flow velocity. Preferably, when operated at said linear flow velocity, the change in porosity of the hydrophilic stationary phase and/or the rigid beads with throughpores is less 50%, preferably less than 40%, preferably less than 30%, preferably less than 20%, preferably less than 10%, most preferably less than 5% compared to the porosity of the hydrophilic stationary phase and/or the rigid beads with throughpores in a fully hydrated state at standard pressure. In preferred embodiments of the invention, prior to step (i) or preferably step y) the stationary phase is equilibrated with the hydrophilic mobile phase comprising the non-ionic polymer. This embodiment guarantees a homogenous distribution of the non-ionic polymer in the stationary phase during step (i) or preferably step y) and may for example be used to minimize flow-through of target nanoparticles.

In preferred embodiments of the invention, prior to step (i) or preferably step y) the target nanoparticles are dispersed in the hydrophilic mobile phase, preferably by in-line mixing. Between dispersing the nanoparticles in the hydrophilic mobile phase and conducting step (i) or preferably step y), any time interval may be chosen. Preferably, in this time interval, the target nanoparticles do not self-aggregate. Preferably, "in-line" mixing refers to dispersing the target nanoparticles in the hydrophilic mobile phase in a vessel, tubing or inlet before the hydrophilic stationary phase.

In the context of the chromatography system, "in-line mixing" may refer to dispersing the target nanoparticles in the hydrophilic mobile phase in a module before the module containing the hydrophilic stationary phase. In alternative embodiments, "in-line mixing" refers to dispersing the target nanoparticles in the hydrophilic mobile phase in the same module as the module containing the hydrophilic stationary phase.

In preferred embodiments, dispersing the target nanoparticles in the hydrophilic mobile phase is conducted less than 50 ms, preferably less than 40 ms, preferably less than 30 ms, preferably less than 20 ms, preferably less than 10 ms, or most preferably less than 5 ms prior to step (i) or preferably step y). In-line mixing allows to reduce the time, during which the target nanoparticles are dispersed in the hydrophilic mobile phase. Furthermore, in-line mixing allows changing parameters during the subsequent loading phase of step (i) or preferably step y) using a gradient. Preferably, these parameters are parameters of the target nanoparticles (such as the target nanoparticle concentration and/or type of targe nanoparticles) and/or parameters of the hydrophilic mobile phase (such as the solvent, buffer, pH, ion type, ion concentration, ionic strength, type of non-ionic dissolved polymer and/or concentration of the non-ionic dissolved polymer) during loading.

In preferred embodiments of the invention, the target nanoparticles and the hydrophilic mobile phase are fed to the stationary phase in separate inlets. By doing so, the time during which the target nanoparticles are dispersed in the hydrophilic mobile phase is minimized. This is particularly advantageous, if the target-nanoparticles aggregate, when dispersed in the hydrophilic mobile phase.

In preferred embodiments of the invention, the rigid beads with throughpores feature a mean bead diameter of 3 µm to 300 µm, preferably 3 µm to 200 µm, preferably 3 µm to 100 µm., preferably, the rigid beads with throughpores feature a mean bead diameter of 3 µm to 40 µm, preferably 3 µm to 30 µm, preferably 3 µm to 20 µm, preferably 3 µm to 15 µm. In the context of the invention, the mean bead diameter refers to beads in a dried state. Preferably, the mean bead diameter is determined by scanning electron microscopy. In particularly preferred examples, the mean bead diameter may be determined on rigid beads with throughpores that have previously been dried from an ethanol solution at 50 °C at 10⁻³ mbar using a Helios G3 UC (*FEI*) scanning electron microscope that is operated at 3kV using a through-lens detector, preferably wherein the rigid beads with throughpores are carbon sputtered and placed on a carbon film that is fixed on an aluminium sample holder.

Throughout the application, the rigid beads with throughpores are on some occasions referred to as "spherical". However, the shape of the rigid beads with throughpores of the invention is not generally limited to a sphere. The rigid beads with throughpores can be in any shape. In case of non-spherical rigid beads with throughpores, when referring to a bead diameter, the bead diameter preferably refers to the longest distance between two points that are within the volume of a single rigid bead.

"Porosity" in the context of the invention refers to the fraction of the volume in the pores of a material over the total volume of the material. For example, if the stationary phase is in the shape of a cylinder, porosity refers to the ratio of the volume in the pores of the stationary phase divided by the volume of the cylinder. For example, if referring to the porosity of a spherical rigid bead with throughpores, the porosity of the spherical rigid bead with throughpores is the ratio of the volume of the pores of the rigid bead with throughpores and the volume of the sphere.

In the context of the invention, the porosity may be determined by mercury porosimetry or by nitrogen sorption. Preferably, these methods are conducted on a sample that is dried from an ethanol solution at 50 °C at 10⁻³ mbar and can be conducted with any suitable device. In a particularly preferred example, the porosity is determined using a mercury intrusion porosimeter *(porous materials* inc.) on a sample that is dried from an ethanol solution at 50 °C at 10⁻³ mbar. In another particularly preferred example, the porosity is determined using a Nova 600 *(Anton Paar)* on a sample that is dried from an ethanol solution at 50 °C at 10⁻³ mbar.

Throughout the invention various kinds of pores are mentioned. A pore is a void in a material that can be empty or filled, for example with another phase, with target nanoparticles and/or with solvent. The invention differentiates between various subsets of pores: interstitial pores, diffusive pores and throughpores. The hydrophilic stationary phase according to the invention comprises throughpores. Preferably the hydrophilic stationary phase further comprises diffusive pores and/or interstitial pores. Preferably, the hydrophilic stationary phase comprises throughpores, diffuse pores and interstitial pores.

The rigid beads with throughpores of the present invention, for example if the rigid beads with throughpores are all spherical, do not completely fill up the whole volume of the of the stationary phase. In-between the individual rigid beads with throughpores of the stationary phase, there are voids. These voids are referred to as "interstitial pores".

A "throughpore" in the context of the invention refers to a channel that transects a bead such as the rigid beads of the invention. Such a throughpore has at least one entrance into the bead and at least one exit out of the bead. The throughpores of the rigid beads of the invention may provide beneficial properties such as an increased surface area that is accessible to the target nanoparticles, convective mass transport into the pores of a bead, facilitation of diffusive mass transport, and provide the rigid beads with a semi convective flow character. Preferably, a rigid bead with throughpores comprises at least one throughpore.

In preferred embodiments of the invention, the throughpores exhibit a mean throughpore diameter of at least 0.02%, preferably at least 0.2% of the mean bead diameter. Preferably, the throughpores exhibit a mean throughpore diameter of at least 0.02% of the mean bead diameter, and preferably at least 10 nm. More preferably, the throughpores exhibit a mean throughpore diameter of at least 0.2% of the mean bead diameter, and preferably at least 50 nm. Preferably, the throughpores exhibit a mean throughpore diameter of at least 10 nm, preferably at least 50 nm, preferably at least 100 nm, preferably at least 150 nm, preferably at least 200 nm, preferably at least 400 nm, preferably at least 600 nm, most preferably at least 800 nm. In preferred embodiments of the invention, the throughpores exhibit a mean throughpore diameter that is smaller than 17%, preferably smaller than 13%, preferably smaller than 10%, most preferably smaller than 8% of the mean bead diameter.

In preferred embodiments of the invention, the throughpores exhibit a mean throughpore diameter of 0.02% to 17%, more preferably 0.02% to 13%, more preferably 0.02% to 10%, most preferably 0.02% to 8% of the mean bead diameter, and preferably wherein the throughpore diameter is at least 10 nm. Preferably, the throughpores exhibit a mean throughpore diameter of 0.2% to 17%, more preferably 0.2% to 13%, more preferably 0.2% to 10%, most preferably 0.2% to 8% of the mean bead diameter, and preferably wherein the mean throughpore diameter is at least 50 nm.

In preferred embodiments of the invention, the throughpores exhibit a mean throughpore diameter of 10 nm to 500 nm, preferably 50 nm to 500 nm, preferably 100 nm to 500 nm, preferably 150 nm to 500 nm, preferably 100 nm to 400 nm, preferably 200 nm to 400 nm, most preferably 150 nm to 350 nm.

In the context of the invention, the mean throughpore diameter may be determined by mercury porosimetry or by nitrogen sorption. Preferably, these methods are conducted on a sample that is dried from an ethanol solution at 50 °C at 10⁻³ mbar and can be conducted with any suitable device. In a particularly preferred example, the mean throughpore diameter is determined using a mercury intrusion porosimeter *(porous materials* inc.) on a sample that is dried from an ethanol solution at 50 °C at 10⁻³ mbar. In another particularly preferred example, the mean throughpore diameter is determined using a Nova 600 *(Anton Paar)* on a sample that is dried from an ethanol solution at 50 °C at 10⁻³ mbar.

In preferred embodiments of the invention, the rigid beads with throughpores comprise diffusion pores. "Diffusion pores" in the context of the rigid beads with throughpores refers to a subset of pores that does not transect the bead. Target nanoparticles and/or impurities can diffuse into such diffusion pores. Preferably, the diffusion pores comprise a mean diffusion pore diameter that is smaller than the mean throughpore diameter. Preferably, the diffusion pores comprise a mean diffusion pore diameter of 5 to 200 nm, more preferably 5 to 150 nm.

In the context of the invention, the mean diffusion pore diameter may be determined by mercury porosimetry or by nitrogen sorption. Preferably, these methods are conducted on a sample that is dried from an ethanol solution at 50 °C at 10⁻³ mbar and can be conducted with any suitable device. In a particularly preferred example, the mean diffusion pore diameter is determined using a mercury intrusion porosimeter *(porous materials* inc.) on a sample that is dried from an ethanol solution at 50 °C at 10⁻³ mbar. In another particularly preferred example, the mean diffusion pore diameter is determined using a Nova 600 *(Anton Paar)* on a sample that is dried from an ethanol solution at 50 °C at 10⁻³ mbar.

Pores in the present invention can be isolated, such as in the case of a single throughpore that penetrates a rigid bead with throughpores, or connected, for example in a pore network. Preferably, a throughpore is connected to at least one other throughpore. Preferably, a throughpore is connected to at least one diffusion pore. Furthermore, a diffusion pore and/or a throughpore can be connected to at least one interstitial pore.

In preferred embodiments of the invention, the rigid beads with throughpores contain a rigid material.

In this context, "rigidity" preferably refers to one or more of the following:
- the rigid material, when packed in a chromatography column with a chromatography column height of 30 cm and using a linear flow velocity of 350 cm/h, exhibits a volume change of less than 50%, preferably less than 40%, preferably less than 30%, preferably less than 20%, preferably less than 10%, most preferably less than 5% compared to its fully hydrated volume at standard pressure;
- the rigid material, when packed in a chromatography column with a chromatography column height of 30 cm and using a linear flow velocity of 350 cm/h, exhibits a change in its porosity of less than 50%, preferably less than 40%, preferably less than 30%, preferably less than 20%, preferably less than 10%, most preferably less than 5% compared to its porosity in a fully hydrated state at standard pressure;
- the rigid material withstands a pressure of at least 10 bar, preferably at least 60 bar, preferably at least 100 bar, preferably at least 200 bar, most preferably at least 300 bar.

In this context, "withstanding" may refer to a volume change of less than 50%, preferably less than 40%, preferably less than 30%, preferably less than 20%, preferably less than 10%, most preferably less than 5% compared to the fully hydrated volume of the rigid material at standard pressure. Alternatively, in this context, "withstanding" may refer to a change in porosity of less than 50%, preferably less than 40%, preferably less than 30%, preferably less than 20%, preferably less than 10%, most preferably less than 5% of the rigid material compared to its porosity in a fully hydrated state at standard pressure.

In further alternative embodiments of the invention, the parameters regarding the "rigidity" of the rigid material, the rigid hydrophilic stationary phase, and/or the rigid beads refer to a linear flow speed of 500 cm/h, preferably 900 cm/h, preferably 2000 cm/h.

Therefore, in preferred further alternative embodiments, "rigidity" refers to one or more of the following:
- the rigid material, the hydrophilic stationary phase and/or the rigid beads with throughpores, when packed in a chromatography column with a height of 30 cm and using a linear flow velocity of 900 cm/h, exhibit a volume change of less than 50%, preferably less than 40%, preferably less than 30%, preferably less than 20%, preferably less than 10%, most preferably less than 5% compared to their fully hydrated volume at standard pressure;
- the rigid material, the hydrophilic stationary phase and/or the rigid beads with throughpores, when packed in a chromatography column with a chromatography column height of 30 cm and using a linear flow velocity of 900 cm/h, exhibit a change in their porosity of less than 50%, preferably less than 40%, preferably less than 30%, preferably less than 20%, preferably less than 10%, most preferably less than 5% compared to their porosity in a fully hydrated state at standard pressure;
- the rigid material, the hydrophilic stationary phase and/or the rigid beads with throughpores withstand a pressure of at least 10 bar, preferably at least 60 bar, preferably at least 100 bar, preferably at least 200 bar, most preferably at least 300 bar.

Furthermore, in preferred further alternative embodiments, "rigidity" refers to one or more of the following:
- the rigid material, the hydrophilic stationary phase and/or the rigid beads with throughpores, when packed in a chromatography column with a height of 30 cm and using a linear flow velocity of 900 cm/h, exhibit a volume change of less than 50%, preferably less than 40%, preferably less than 30%, preferably less than 20%, preferably less than 10%, most preferably less than 5% compared to their fully hydrated volume at standard pressure;
- the rigid material, the hydrophilic stationary phase and/or the rigid beads with throughpores, when packed in a chromatography column with a chromatography column height of 30 cm and using a linear flow velocity of 900 cm/h, exhibit a change in their porosity of less than 50%, preferably less than 40%, preferably less than 30%, preferably less than 20%, preferably less than 10%, most preferably less than 5% compared to their porosity in a fully hydrated state at standard pressure;
- the rigid material, the hydrophilic stationary phase and/or the rigid beads with throughpores withstand a pressure of at least 10 bar, preferably at least 60 bar, preferably at least 100 bar, preferably at least 200 bar, most preferably at least 300 bar.

Furthermore, in preferred further alternative embodiments, "rigidity" refers to one or more of the following:
- the rigid material, the hydrophilic stationary phase and/or the rigid beads with throughpores, when packed in a chromatography column with a height of 30 cm and using a linear flow velocity of 2000 cm/h, exhibit a volume change of less than 50%, preferably less than 40%, preferably less than 30%, preferably less than 20%, preferably less than 10%, most preferably less than 5% compared to their fully hydrated volume at standard pressure;
- the rigid material, the hydrophilic stationary phase and/or the rigid beads with throughpores, when packed in a chromatography column with a chromatography column height of 30 cm and using a linear flow velocity of 2000 cm/h, exhibit a change in their porosity of less than 50%, preferably less than 40%, preferably less than 30%, preferably less than 20%, preferably less than 10%, most preferably less than 5% compared to their porosity in a fully hydrated state at standard pressure;
- the rigid material, the hydrophilic stationary phase and/or the rigid beads with throughpores withstand a pressure of at least 10 bar, preferably at least 60 bar, preferably at least 100 bar, preferably at least 200 bar, most preferably at least 300 bar.

The above further alternative embodiments correlate the volume change or the porosity change of the hydrophilic stationary phase or the rigid beads with throughpores, when packed in a chromatography column with a chromatography column height of 30 cm and using a linear flow velocity of 500 cm/h, preferably 900 cm/h, preferably 2000 cm/h, with the respective features of the rigid material, the hydrophilic stationary phase or the rigid beads with throughpores, when fully hydrated at standard pressure. Said embodiments are meant to disclose general thresholds to the features of the rigid beads with throughpores, the rigid material, or the hydrophilic stationary phase. They do not limit the chromatography method to be operated using a chromatography column, a flow velocity of 500 cm/h, preferably 900 cm/h, preferably 2000 cm/h. and a chromatography column height of 30 cm and they do not limit the chromatography method to be conducted at a fully hydrated state of the rigid material, rigid beads with throughpores or the hydrophilic stationary phase at standard pressure.

In preferred embodiments of the invention, the rigid material is a ceramic, a silica, a metal-organic framework, a polymer, a coordination polymer, a covalent organic framework, a zeolite, an alumina or a combination thereof. In preferred embodiments of the invention, the polymer is a polystyrene divinylbenzene (PS/DVB).

The hydrophilic stationary phase of the present invention comprises rigid beads with throughpores. Preferably, the rigid beads with throughpores according to the invention comprise a "backbone". In the context of the invention, the term "backbone" refers to a material, out of which a rigid bead may be fully or partially be composed of, that determines the three-dimensional shape of the rigid bead. Preferably, the backbone is a rigid material according to any of the embodiments of the invention.

The stationary phase, the rigid beads with throughpores and the backbone comprise a surface. The surface of the rigid beads with throughpores consists of an external surface and an internal surface. The external surface is the interface of the rigid beads with throughpores with the medium that they are surrounded with. The internal surface is the interface of the rigid beads with throughpores with the medium that they are permeated with. This internal surface corresponds to the surface of the pores of the rigid bead with throughpores. Preferably, the external surface of the rigid beads with throughpores corresponds to the external surface of the stationary phase. Preferably, the internal surface of the rigid beads with throughpores corresponds to the internal surface of the stationary phase.

The invention relies on a hydrophilic stationary phase. However, it is not necessary that the entire stationary phase consists of a single hydrophilic material. In preferred embodiments of the invention, the hydrophilic stationary phase exhibits a hydrophilic surface. In the context of the invention, the term "hydrophilic" is used for a material, a surface, a functional group, or a molecule that interacts thermodynamically more favourable with water and other polar substances than with oil and other hydrophobic substances. Without wishing to be bound by theory, a hydrophilic material, surface, backbone, functional group or molecule is also considered polar. Preferably, the rigid beads with throughpores comprise a hydrophilic surface. Preferably, the backbone comprises a hydrophilic surface.

In preferred embodiments of the invention, the hydrophilic surface, preferably the internal and/or external surface of the rigid beads with throughpores, compromises at least one type of hydrophilic surface group. Preferably, the backbone comprises at least one hydrophilic surface group. Preferably, the at least one type of hydrophilic surface group is selected from epoxide group, carbonyl group, aldehyde group, thiol group, lactone group, acid anhydride group, amine group, carboxyl group, sulfonic acid group, phosphate ester group, and phosphonic acid group.

In preferred embodiments of the invention, the hydrophilic surface group is selected from epoxide group, carbonyl group, aldehyde group, thiol group, lactone group, acid anhydride group.

In preferred embodiments of the invention, the hydrophilic surface group is a cation exchange group, preferably the cation exchange group is selected from carboxyl group (preferably carboxymethyl group), sulfonic acid group (preferably sulfomethyl group and sulfopropyl group), phosphate ester group and phosphonic acid group.

Preferably, the hydrophilic stationary phase of the invention is surface-functionalized. Preferably, the internal and/or the external surface of the rigid beads with throughpores is surface-functionalized. In preferred embodiments of the invention, the rigid beads with throughpores contain a backbone that is surface-functionalized. In preferred embodiments of the invention the hydrophilic surface group is bound directly to the surface of the hydrophilic stationary phase, the external and/or internal surface of the rigid beads, or the hydrophilic surface group is bound to said surfaces with a bridging (CH₂)₁₋₃ alkyl, as for example in carboxymethyl group, sulfomethyl group or sulfopropyl group. Preferably, the hydrophilic surface group is directly bound to the backbone of the rigid beads with throughpores, or the hydrophilic surface group is bound to the backbone of the rigid beads with throughpores with a bridging (CH₂)₁₋₃ alkyl, as for example in carboxymethyl group, sulfomethyl group or sulfopropyl group.

In optional step (ii) or step y'), the hydrophilic stationary phase is washed with a hydrophilic washing phase that preferably also comprises a non-ionic dissolved polymer. Step (ii) or preferably step y') is conducted after conducting step (i) or preferably step y). Thus, the target nanoparticles are attached to the surface of the hydrophilic stationary phase. Preferably, during the optional step (ii) or preferably step y'), the target nanoparticles stay attached to the surface of the stationary phase. Preferably, this is achieved by the non-ionic dissolved polymer that is comprised in the hydrophilic washing phase. In preferred embodiments of the invention, the non-ionic dissolved polymer of step (i) or preferably step y) and step (ii) or preferably step y') are the same non-ionic dissolved polymer. In preferred embodiments of the invention, hydrophilic washing phase is the hydrophilic mobile phase.

Preferably, prior to step (i) or preferably step y) the target nanoparticles are in a solution and/or a dispersion, such as a suspension. Preferably, the first solution of the one alternative of the first aspect is such a solution and/or dispersion of the target nanoparticles. Preferably, in this context the continuous medium of the solution and/or dispersion is a liquid that is preferably selected from any hydrophilic solvent or hydrophilic solvent mixture. In preferred embodiments of the invention, the liquid comprises water, preferably the liquid is an aqueous buffer or water. Preferably, the liquid is a pharmaceutically acceptable excipient. Preferably, the liquid is a biological solution such as a culture medium, a cell suspension, a lysed cell suspension, and/or a body fluid.

Preferably, the second solution of the one alternative of the first aspect is the hydrophilic mobile phase. In the present invention, the hydrophilic mobile phase, the eluent and the optional hydrophilic washing phase can be selected from any hydrophilic solvent or hydrophilic solvent mixture. In preferred embodiments of the invention, the hydrophilic mobile phase, the eluent and/or the optional hydrophilic washing phase comprises water, preferably the hydrophilic mobile phase, the eluent and/or the optional hydrophilic washing phase is an aqueous buffer or water. Preferably, the eluent, the washing phase and or the hydrophilic mobile phase is a pharmaceutically acceptable excipient.

In preferred embodiments of the invention, the target nanoparticles are macromolecules, preferably biological macromolecules including extracellular vesicles (such as exosomes), liposomes, mitochondria, chloroplasts, lysosomes and/or other cellular organelles, viruses, virus-like particles, viral gene vectors or proteins such as antibodies. Preferably, the target nanoparticles are viruses, virus-like particles, viral gene vectors or proteins such as antibodies. Preferably, the target nanoparticles, for example the viruses, the viral gene vectors or the virus-like particles, comprise an average number-based hydrodynamic diameter that is larger than 300 nm, preferably that is larger than 200 nm, 100 nm, 50 nm, 40 nm, 30 nm, 20 nm or 10 nm, most preferably 10 nm. Preferably, the hydrodynamic diameter is determined by dynamic light scattering. In one particular example, the hydrodynamic diameter may be determined using a Zetasizer Advance *(Malvern Panalytical GmbH)* in an aqueous such as a 50 mM TRIS, 150 mM NaCl buffer. If dynamic light scattering is not suitable to determine the hydrodynamic radius of the virus particles, a person skilled in the art might use other methods such as differential centrifugal sedimentation (DCS), multi-angle light scattering (MALS), and/or tunable resistive pulse sensing (TRPS).

In preferred embodiments of the invention, the viruses have an empty capsid and/or a capsid that is not empty. In case the viruses have an empty capsid and a capsid that is not empty, this refers to a mixture of virus particles with empty capsids and capsids that are not empty.

In preferred embodiments, the virus is selected from any virus of the groups of dsDNA virus (such as adenovirus, herpesvirus, poxyvirus), ssDNA virus (such as parvovirus), dsRNA virus (such as reovirus), (+)ssRNA virus (such as coronavirus, hepatitis C virus, picornavirus and togavirus), (-)ssRNA virus (such as orthomyxovirus and rhabdovirus), ssRNA-RT virus (such as retrovirus) and a dsDNA-RT virus (such as hepadnavirus).

Virus-like particles resemble viruses but are non-infectious because they do not contain viral genetic material. Preferably, they comprise viral structural proteins such as envelope or capsule proteins.

In preferred embodiments of the invention, the target nanoparticles is a mixture of at least two types of target nanoparticles. "Type" in the context of the target nanoparticles, refers to a difference in the physical, chemical and/or biological properties such as weight or chemical structure. Preferably, the target nanoparticles is a mixture of a first type of virus and a second type of virus. Preferably, the virus is *Severe acute respiratory syndrome coronavirus type 2, Human immunodeficiency virus* and/or *Hepatitis C virus.* Preferably, the target nanoparticles is a mixture of a viruses and virus-like particles. Preferably, the target nanoparticles is a mixture of viruses that have an empty capsid and viruses that have a capsid that is not empty. Preferably, the target nanoparticles is mixture of a first type of target protein and a second type of target protein.

Without wishing to be bound by theory, the chromatography method of the present invention relies on unfavourable free energy that results from the steric exclusion of the non-ionic dissolved polymer from the target nanoparticles. This free energy stems from deficiency zones in the solvent that is amongst others dependent on the hydrodynamic diameter of the non-ionic dissolved polymer. With increasing hydrodynamic diameter of the non-ionic dissolved polymer, the effect that the non-ionic dissolved polymer exerts on the thermodynamic system increases. Amongst others, the hydrodynamic diameter of the non-ionic dissolved polymer is dependent on the molecular structure of the non-ionic dissolved polymer. In preferred embodiments of the invention, the non-ionic dissolved polymer is a polyol or a poloxamer, preferably the non-ionic dissolved polymer is a polyalkylene glycol, preferably having two or three carbon atoms in the repeating unit, more preferably, a polyethylene glycol (PEG), a polypropylene glycol or mixture thereof.

In preferred embodiments, the non-ionic dissolved polymer is a non-ionic derivative of the non-ionic dissolved polymer, preferably the PEG. In preferred embodiments of the invention, the non-ionic dissolved polymer, such as the PEG, features a linear, branched (preferably comb-shaped) or star geometry. These geometries refer to constitutional isomers of the non-ionic dissolved polymer. In star geometry, 4 to 8 arms, preferably 4, 6, or 8 arms of a linear non-ionic polymer are connected to a single internal point. Comb geometry refers to a branched geometry, wherein multiple chains are grafted onto a polymer chain in repeating intervals to result in a comb-like appearance.

Furthermore, the hydrodynamic diameter of the non-ionic dissolved polymer increases with the size of the non-ionic dissolved polymer. In preferred embodiments of the invention, the non-ionic dissolved polymer is a polyethylene glycol (PEG), preferably wherein the PEG is PEG 2000 to PEG 12000, preferably wherein the PEG is PEG 4000 to PEG 10000, more preferably wherein the PEG is PEG 5000 to PEG 8000, most preferably wherein the PEG is PEG 6000.

The free energy associated with the exclusion of the non-ionic dissolved polymer from the surface of the target nanoparticles is depending on the concentration of the non-ionic dissolved polymer. In preferred embodiments, the non-ionic dissolved polymer in the hydrophilic mobile phase is dissolved in a mass fraction of 1% to 40%, preferably 2% to 20%, more preferably 4% to 12%, most preferably 6% to 10%.

After conducting step (i) or preferably step y), the target nanoparticles are attached to the surface of the hydrophilic stationary phase. In step (iii) or preferably step z), the target nanoparticles are eluted from the hydrophilic stationary phase with an eluent. Without wishing to be bound by theory, step (iii) or preferably z) creates a new thermodynamic system, in which the eluent favours the target nanoparticles to be situated in the eluent more strongly than the hydrophilic mobile phase during loading in step (i) or preferably y). Step (iii) or preferably z) results in the target nanoparticles being detached from the surface the hydrophilic stationary phase. After conducting step (iii) or preferably z), the eluent exiting the hydrophilic stationary phase comprises the target nanoparticles. Consequently, conducting step (iii) or preferably z) leads to an elution of the target nanoparticles.

Preferably, the eluent does not comprise a non-ionic dissolved polymer. However, the methods can still be performed using an eluent that comprises a non-ionic dissolved polymer.

In preferred embodiments of the invention, the eluent comprises a non-ionic dissolved polymer. Preferably, the non-ionic dissolved polymer is a non-ionic dissolved polymer according to any of the embodiments of the invention. In preferred embodiments of the invention, the non-ionic dissolved polymer in the eluent and the non-ionic dissolved polymer in the hydrophilic mobile phase are the same polymer. Preferably, the eluent comprises at least two types of dissolved polymers.

The free energy of the thermodynamic system is depending on the concentration of the solutes that are sterically excluded. A higher solute concentration means a higher difference in solute concentration in the deficiency zones compared to the solute concentration in the bulk. In preferred embodiments of the invention, the eluent comprises a different mass fraction of the non-ionic dissolved polymer in the eluent compared to the mass fraction of the non-ionic dissolved polymer in the hydrophilic mobile phase, preferably wherein the mass fraction of the non-ionic dissolved polymer in the eluent is smaller.

Without wishing to be bound by theory, the free energy associated with the exclusion of the non-ionic dissolved non-ionic polymer from the surface of the target nanoparticles is depending on the ion-concentration and/or ionic strength of the system. A high ion-concentration and/or ionic strength can lead to a decreased hydrodynamic radius of the non-ionic dissolved polymer, as it results in a more compact coiled structure of the non-ionic dissolved polymer. Consequently, the steric exclusion effect of the non-ionic dissolved polymer is decreased with higher ion-concentrations and/or ionic strength.

This effect can also be made use of when conducting step (iii) or preferably step z). In preferred embodiments, the eluent comprises a higher ionic strength and/or a higher ion-concentration in the eluent compared to the ionic strength and/or ion-concentration of the hydrophilic mobile phase. In preferred embodiments, the eluent comprises a higher salt concentration in the eluent compared to the salt concentration of the hydrophilic mobile phase.

Preferably, the salt is a salt used in a biological buffer. In preferred embodiments of the invention, the salt is selected from the Hofmeister series, also known as lyotropic series. Most preferably, the salt is sodium chloride and/or ammonium chloride. In preferred embodiments of the invention, the eluent has a different pH, preferably a higher pH, compared to the pH in the hydrophilic mobile phase.

In preferred embodiments of the invention the target nanoparticles are detached in multiple fractions. For example, if the target nanoparticles is at least two types of target nanoparticles, one type of target nanoparticles might be detached first and is then followed by second type of target nanoparticles and so on. In preferred embodiments, after conducting step (iii) or preferably step z), a fraction of target nanoparticles might still be attached to the column-stacked hydrophilic stationary phase in the form of residual target nanoparticles. Preferably, these residual target nanoparticles are detached by repetition of step (iii) or preferably step z), preferably at least two repetitions. In preferred embodiments of the invention, the target nanoparticles are detached in multiple fractions by repetition of step (iii) or preferably step z).

In preferred embodiments of the invention, the target nanoparticles are detached from the hydrophilic stationary phase by step elution, by gradient elution or by a mixture thereof.

In preferred embodiments of the invention, the target nanoparticles are detached from the hydrophilic stationary phase by step elution. When referring to step elution, step (iii) or preferably step z) is preferably repeated at least two times, wherein for each repetition, the eluent, here referred to as "previous eluent", is exchanged with "another eluent". Preferably, the another eluent is the same eluent as the "previous eluent". Preferably, the another eluent comprises a different concentration of the non-ionic dissolved polymer, preferably a lower concentration, compared to the previous eluent. Preferably, the another eluent comprises a different non-ionic dissolved polymer, pH, salt concentration, ionic strength, and/or ion-concentration than the previous eluent. Preferably, the pH, salt concentration, ionic strength and/or ion-concentration of the another eluent is higher than the salt concentration, ionic strength and/or ion-concentration of the previous eluent.

In preferred embodiments of the invention, the target nanoparticles are eluted with an eluent not containing any non-ionic dissolved polymer, or a containing a reduced amount of the non-ionic dissolved polymer compared to the hydrophilic mobile phase and optionally the washing phase. In a preferred embodiment of the invention the elution comprises an elution using a sequence of multiple eluents of decreasing concentration of the non-ionic dissolved polymer. The eluents of such a sequence of eluents may, preferably sequentially, comprise about 80%, about 70%, about 60% (and so on) of the concentration of the non-ionic dissolved polymer compared to the concentration of the non-ionic dissolved polymer used for attaching the target nanoparticles to the stationary phase. In a preferred embodiment of the invention the elution comprises an elution using a sequence of multiple eluents of increasing pH, salt concentration, ionic strength and/or ion-concentration.

In preferred embodiment of the invention, the target nanoparticles are detached from the hydrophilic stationary phase by gradient elution. In such a gradient elution, target nanoparticles that exhibit a similar attachment behaviour to the hydrophilic stationary phase may be detached sequentially with high precision. Furthermore, eluents that are suitable to detach nanoparticles from the surface of the hydrophilic stationary phase can be determined. In preferred embodiments of the invention, the ionic strength and/or ion-concentration and/or salt concentration in the eluent is increasing with time. In preferred embodiments of the invention, the mass fraction of the non-ionic dissolved polymer in the eluent is decreasing with time. In preferred embodiments of the invention, the pH of the eluent is changing with time. Preferably the pH of the eluent is increasing with time.

In **a second aspect,** the invention pertains to a composition comprising purified and/or separated nanoparticles, wherein the purified and/or separated nanoparticles are the target nanoparticles obtainable by the chromatography method according to the previous aspect of the invention.

In preferred embodiments of the invention, the composition comprising the purified and/or separated nanoparticles is the purified and/or separated nanoparticles of the previous aspect of the invention. Preferably, the composition comprising the purified and/or separated nanoparticles is a solid, such as a powder, an emulsion or a solution.

In preferred embodiments of this aspect, the composition comprising purified and/or separated nanoparticles is the eluent or comprises the eluent after conducting step (iii) or preferably step z) of the previous aspect of the invention.

Preferably, the composition comprises the non-ionic dissolved polymer according to any embodiment of the invention. In other embodiments, the non-ionic dissolved polymer may be removed from the eluent after conducting step (iii) or preferably step z) of the previous aspect of the invention.

In preferred embodiments of this aspect, the target nanoparticles, that are contained in the eluent exiting the stationary phase after conducting step (iii) or preferably step z) of the previous aspect of the invention, are separated from the eluent and dispersed in a new solvent. Preferably the new solvent is a biological buffer system and/or a pharmaceutically acceptable excipient.

**In a third aspect,** the invention pertains to a composition comprising purified and/or separated nanoparticles for use in medicine, wherein the composition comprising purified and/or separated nanoparticles is a composition according to the second aspect of the invention.

In **a fourth aspect,** the invention pertains to a composition comprising purified and/or separated nanoparticles for use in the manufacturing of a medicament or a vaccine, wherein the composition comprising purified and/or separated nanoparticles is a composition according to the second aspect of the invention.

In preferred embodiments of the invention, the composition comprising purified and/or separated nanoparticles comprises a pharmaceutically acceptable excipient. Preferably, the eluent, the washing phase and or the hydrophilic mobile phase is the pharmaceutically acceptable excipient.

In **a fifth aspect,** the invention pertains to a method for the depletion of nanoparticles from a liquid target composition, comprising the sequential steps:
(u) dissolving a non-ionic polymer according to any previous aspect of the invention in the liquid target composition in a concentration according to any previous aspect of the invention;
(v) bringing into contact the liquid target composition containing the non-ionic dissolved polymer with a hydrophilic stationary phase according to any previous aspect of the invention, thus allowing the nanoparticles to attach to the hydrophilic stationary phase and creating a nanoparticle-depleted target composition;
(w) eluting the nanoparticle-depleted target composition,
wherein the hydrophilic stationary phase is packed in a chromatography device and the nanoparticles are the target nanoparticles according to any previous aspect of the invention.

**In a sixth aspect,** the invention pertains to a nanoparticle-depleted composition, obtainable by the method for the depletion of nanoparticles according to the fifth aspect of the invention.

Preferably, the nanoparticle-depleted composition is and/or comprises the nanoparticle-depleted target composition of the previous aspect of the invention.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

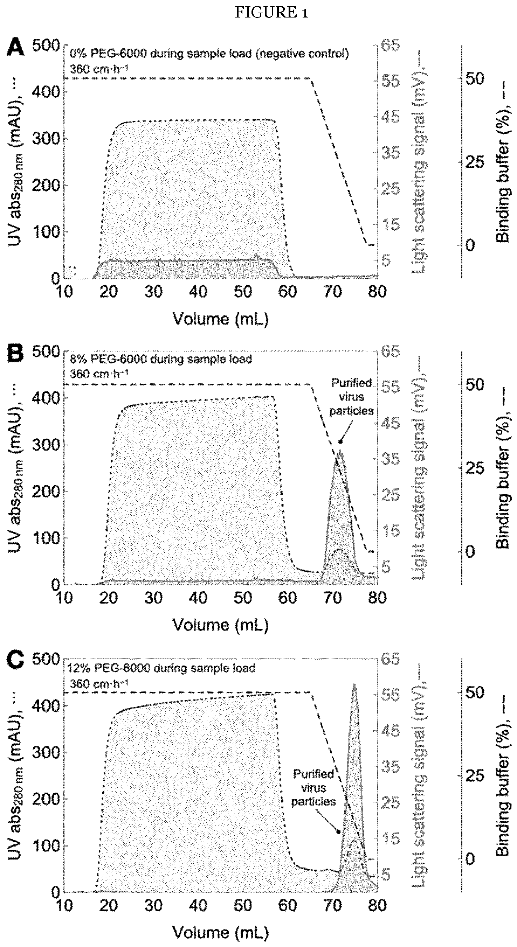

**Figure 1** shows: Three SXC chromatograms showing the purification of an *influenza A virus* containing sample on hydrophilic rigid beads that are functionalized for cation-exchange. Loading was conducted in (A) without PEG (negative control), (B) at 8% PEG 6000 and (C) at 12% PEG 6000. Impurities and virus particles in the flow-through are detected in dependency of the buffer volume passing through the chromatography column using light scattering signal (solid light grey curve) and UV absorbance at 280 nm (dotted black curve).

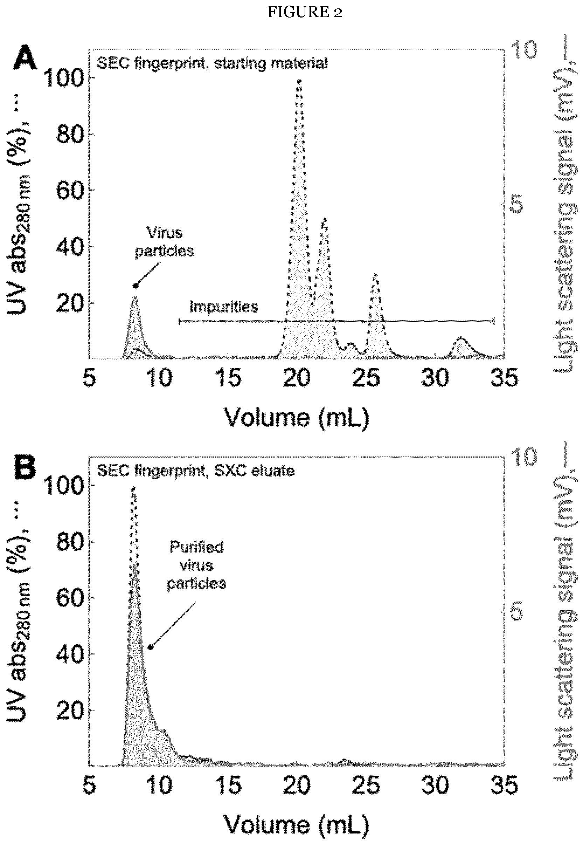

**Figure 2** shows: Analytical SEC fingerprints from (A) the starting material and (B) the SXC eluate from the experiment of Figure 1C, in which loading was conducted at 12% PEG 6000. Impurities and virus particles in the flow-through are detected in dependency of the buffer volume passing through the chromatography column using light scattering signal (solid light grey curve) and UV absorbance at 280 nm (dotted black curve).

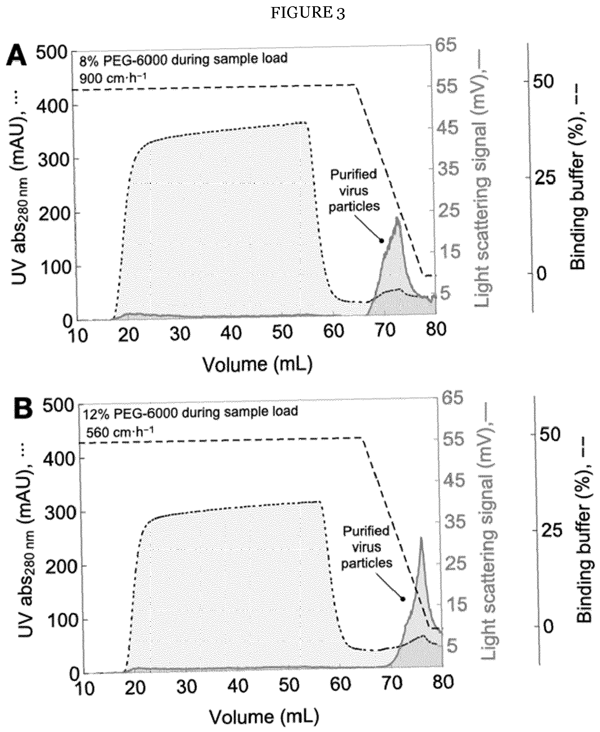

**Figure 3** shows: Two SXC chromatograms showing the purification of an *influenza A virus* containing sample on hydrophilic rigid beads that are functionalized for cation-exchange. (A) linear flow velocity of 900 cm/h and loading at 8% PEG 6000. (B) linear flow velocity of 560 cm/h and loading at 12% PEG 6000. Impurities and virus particles in the flow-through are detected in dependency of the buffer volume passing through the chromatography column using light scattering signal (solid light grey curve) and UV absorbance at 280 nm (dotted black curve).

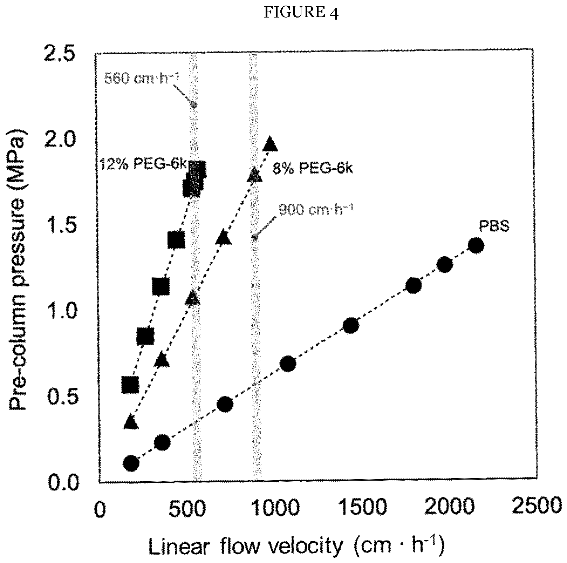

Figure **4** shows: Relationship between pre-column pressure (MPa) and linear flow velocity (cm/h) for the three solvent-systems used during loading in the examples. Grey Lines depict the column pressures in the experimental conditions shown in Example 2.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: SXC on rigid hydrophilic cation exchanger beads

The inventors studied the performance of rigid cation exchanger beads packed in a column for virus purification in SXC. These rigid beads with a diameter of 30 µm and a pore size of 1000 µm feature a PSDVB backbone with throughpores that is functionalized with a very hydrophilic coating, have strong cation exchanger functionality and withstand a pressure of 207 bar. The experiments were performed on cell culture derived and chemically inactivated *influenza A*/*Puerto Rico*/*8*/*34 H1N1 virus* (Robert-Koch Institute, Berlin, Germany).

For this, an AKTA PURE 25 (*Cytiva, Uppsala, Sweden)* liquid chromatography system which is capable of monitoring UV absorption was used. Additionally, the virus particles were detected by light scattering using a *Nicomp 380 Submicron Particle Sizer* in the flow path operating at a wavelength of 640 nm *(Particle sizing systems, Santa Barbara, USA).*

The chromatography experiments of Example 1 were conducted at room temperature using a flow rate of 1 mL/min (corresponding to a linear flow velocity of 360 cm/h).

As indicated by the wide dashed line (Binding buffer (%)) in Figure 1, these experiments were performed by in-line mixing of a binding buffer consisting of 1× PBS, pH 7.4 and a respective PEG 6000 concentration (Figure 1A 0%, Figure 1B 16% and Figure 1C 24%). During equilibration and loading, the sample containing the virus particles (in a ix PBS, pH 7.4, 0% PEG 6000 buffer) was mixed with the binding buffer by in line-mixing prior to the column-packed stationary phase in a 1:1 ratio, resulting in the following PEG 6000 concentrations at which equilibration and loading was conducted: Figure 1A: 0%, Figure 1B: 8%, Figure 1C: 12%).

Figure 1A shows a control experiment, in which the stationary phase was first equilibrated with ix PBS without PEG. Then the column was loaded with the virus sample in a binding buffer consisting of an aqueous solution of ix PBS, pH 7.4. After loading, the device was washed with ix PBS until overall 65 mL of buffer was added to the system.

In the experiment shown in Figure 1B, loading of the column packed stationary phase was performed in an aqueous binding buffer consisting of 1× PBS, 8% PEG 6000, pH 7.4. Prior to loading, the stationary phase, was first equilibrated with 1× PBS, 8% PEG 6000. Subsequently the device was washed with 1 × PBS, 8% PEG 6000 until overall 65 mL of buffer was added to the system.

Analogously, in the experiments shown in Figure 1C, the column-packed stationary phase was first equilibrated with 1 × PBS, 12% PEG 6000. Loading of the column packed stationary phase was performed in an aqueous binding buffer consisting of 1 × PBS, 12% PEG 6000, pH 7.4. After loading, the device was washed with 1 × PBS, 12% PEG 6000 until overall 65 mL of buffer was added to the system.

In all three experiments (Figure 1 A-C), the virus particles were then eluted in the form of a gradient elution from the respective PEG 6000 concentration during loading (Figure 1A: 0%, Figure 1B: 8% and Figure 1C: 16%) to 0% PEG, 1× PBS for 5 CV (1 CV=2.5 mL). The amount of impurities and virus particles leaving the column was detected via light scattering and UV absorbance at 280 nm.

In the control experiments shown in Figure 1A without PEG 6000, the simultaneous increase of scattered light signal and absorbance at 280 nm in the flow-through during loading and washing (18-65 mL Volume) indicates that virus particles substantially failed to attach to the stationary phase without PEG. These virus particles pass the stationary phase together with the impurities. Furthermore, the lack of signal increase during elution shows that no virus particles are detached from the stationary phase.

In the experiments shown in Figure 1B (loading with 8% PEG 6000), the increased UV absorbance at 280 nm demonstrates impurities in the flow through during loading and washing (18-65 mL Volume). At the same time, the light scattering signal that is almost at baseline indicates that the virus particles are attached to the column. During the first elution, both UV and light scattering signal show a peak between 68-75 mL Volume, demonstrating the detachment of the virus particles from the rigid bead stationary phase.

Figure 1C (loading with 12% PEG 6000) gives similar results. In said experiments, an increased UV absorbance between 18-65 mL Volume can be monitored without increase in scattering signal. This increased UV absorbance is caused by impurities that to not attach to the stationary phase and pass the column. The absence of an increased scattering signal between 18-65 mL demonstrates that most of virus particles attach to the surface of the stationary phase. During elution, a combined increase in scattering signal and UV absorbance at 70-77 mL Volume shows that virus particles are detached from the stationary phase.

In the samples loaded with presence of PEG 6000, the elution of the particles was triggered by the eluent having a decreasing concentration of PEG 6000. Furthermore, virus particles failed to attach to the rigid beads without any PEG 6000 in the mobile phase. This proves that the mechanism, by which the virus particles are attached to the cation exchanger stationary phase, is based on SXC.

The inventors then quantified the product recovery of the experiments shown in Figure 1B and Figure 1C by hemagglutination activity (aHA) assay. In the experiments with 8% PEG 6000 concentration during loading, the recovery of virus particles in comparison to the amount of virus particles in the buffer during loading was 43.7%. In case of the experiments with 12% PEG 6000 concentration during loading, a product recovery of even 73.8% was achieved. The higher product recovery can be explained by an increased fraction of virus particles attaching to the stationary phase in case of loading with the higher PEG concentration.

Furthermore, analytical size exclusion chromatography (SEC) fingerprints from the starting material (Figure 2A) and the SXC purified virus particles (Figure 2B) have been determined. In these experiments, a SEC column was used to purify either the starting material (virus sample before being loaded onto the stationary phase) of the prior SXC experiments or the purified virus sample from the eluent of the prior SCX experiments shown in Figure 1C. The flow-through passing the SEC column was analyzed for UV absorbance at 280 nm and for light scattering signal.

In case of the starting material, virus particles exit the column in the range of 7.5-10 mL Volume as indicated by the increased light scattering. A large fraction of impurities can be detected at 19-35 mL Volume from the UV absorbance at 280 nm. In case of the SXC purified sample, no such impurities can be detected. In this sample, both the corresponding sharp light-scattering signal and the UV absorbance at 280 nm show that any impurities have completely been removed.

### Example 2: SXC on rigid hydrophilic cation exchanger beads at high flow velocities

In a further experiment, the inventors tested the compatibility of the rigid hydrophilic cation exchanger beads with faster linear flow velocities.

The corresponding experiments were conducted using the same cation exchanger beads, chromatography system as in Example 1 and equilibration, loading and elution were performed analogously, therefore using in-line mixing, as described above for Example 1. However, the system was operated at faster linear flow velocities.

In the experiment shown in Figure 3A, the stationary phase, was first equilibrated with an aqueous ix PBS, 8% PEG 6000 buffer. Loading of the column packed stationary phase with the sample containing virus particles was performed in an aqueous binding buffer consisting of 1× PBS, 8% PEG 6000, pH 7.4. Subsequently the device was washed with 1× PBS, 8% PEG 6000 until overall 65 mL of buffer was added to the system. The virus particles were then eluted in the form of a gradient elution from the 8% PEG 6000 concentration to 0% PEG, 1× PBS for 5 CV (1 CV=2.5 mL). This experiment was performed at a linear flow velocity of 900 cm/h.

Similarly, Figure 3B shows data of an experiment, in which the column-packed stationary phase was first equilibrated with at 1× PBS, 12% PEG 6000, pH 7.4, and then loaded with a sample containing virus particles at 1× PBS, 12% PEG 6000, pH 7.4 and washed with 1× PBS, 12% PEG 6000, pH 7.4, until overall 65 mL of buffer was added to the system. The virus particles were then eluted using a gradient elution from the 12% PEG 6000 concentration to 0% PEG, 1× PBS for 5 CV (1 CV=2.5 mL). In this experiment, the linear flow velocity was set at 560 cm/h.

Both of these experiments show a successful attachment of virus particles to the stationary phase during loading, which can be seen from the absence of a light scattering signal between, 18-65 mL Volume. On the other hand, impurities pass through the stationary phase during the loading phase, as evident from the increased UV-absorbance. In the subsequent elution, the decreasing PEG concentration in both cases led to a detachment of virus-particles from the hydrophilic stationary phase. For the experiment performed at 8% PEG 6000, an increase in scattering signal in the range of 65-80 mL Volume was detected and indicates the elution of the virus particles. The experiment with a loading at 12% PEG showed a detachment of virus particles between 70-80 mL Volume. Both of these experiments prove that SXC according the present invention using a hydrophilic stationary phase that is based on beads with throughpores can be performed at high speeds.

Furthermore, the inventors screened the amount of pre-column pressure that is generated for the solvent systems the present examples in dependency of linear flow velocity (see Figure 4) Over the whole observed range, column pressure is increasing with increasing linear flow velocity in a linear manner. The slope, by which the pre-column pressure is increasing, varies with the PEG concentration, presumably due to the increased viscosity of the solvent system.

## Claims

1. **A chromatography method for the purification and/or separation of target nanoparticles** comprising the steps of:
(i) loading the target nanoparticles onto a column-packed hydrophilic stationary phase that comprises rigid beads with throughpores in the presence of a hydrophilic mobile phase that comprises a non-ionic dissolved polymer, thus attaching the target nanoparticles to the column-packed hydrophilic stationary phase;
(ii) optionally, washing the column-packed hydrophilic stationary phase with a hydrophilic washing phase that preferably also comprises a non-ionic dissolved polymer;
(iii) eluting the target nanoparticles from the column-packed hydrophilic stationary phase with an eluent;
wherein the hydrophilic stationary phase is packed in a chromatography device;
wherein, the rigid beads with throughpores, when packed in a chromatography column with a chromatography column height of 30 cm and using a linear flow velocity of 350 cm/h, exhibit a volume change of less than 50%, compared to their fully hydrated volume at standard pressure;
optionally, wherein the target nanoparticles are detached in multiple fractions, preferably by repetition of step (iii).

2. The chromatography method according to claim 1, wherein the rigid beads with throughpores, are non-gel rigid beads with throughpores, preferably wherein the rigid beads with throughpores when fully hydrated, comprise an increase in their volume of less than 50%, preferably less than 40%, most preferably less than 30%, when compared to the rigid beads with throughpores in a dried state and/or wherein the rigid beads with throughpores, when using a linear flow velocity of 350 cm/h and a chromatography column height of 30 cm, exhibit a change in their porosity of less than 50%, preferably less than 40%, preferably less than 30%, preferably less than 20%, preferably less than 10%, most preferably less than 5% compared to their porosity in a fully hydrated state at standard pressure.

3. The chromatography method of any one of claims 1 or 2, wherein the rigid beads with throughpores feature a mean bead diameter of 3 µm to 300 µm, preferably 3 µm to 200 µm, preferably 3 µm to 100 µm, preferably 3 µm to 40 µm, 3 µm to 30 µm, 3 µm to 20 µm, or 3 µm to 15 µm, as determined by scanning electron microscopy and/or wherein the throughpores exhibit a mean throughpore diameter of 10 to 500 nm, preferably 50 nm to 500 nm, preferably 100 nm to 500 nm, preferably 150 nm to 500 nm, preferably 100 nm to 400 nm, preferably 200 nm to 400 nm, most preferably 150 nm to 350 nm, as determined by mercury porosimetry.

4. The chromatography method of any one of claims 1 to 3, wherein the rigid beads with throughpores contain a rigid material, preferably wherein the rigid material is a ceramic, a silica, a metal-organic framework, a polymer, a coordination polymer, a covalent organic framework, a zeolite, an alumina or a combination thereof, preferably a polystyrene divinylbenzene (PS/DVB), and/or wherein the rigid beads with throughpores contain a backbone that is surface-functionalized, preferably wherein the backbone is the rigid material.

5. The chromatography method of any one of claims 1 to 4, wherein the column-packed hydrophilic stationary phase exhibits a hydrophilic surface, preferably, wherein the hydrophilic surface comprises at least one type of hydrophilic surface group, preferably wherein the at least one type of hydrophilic surface group is selected from epoxide group, carbonyl group, aldehyde group, thiol group, lactone group, acid anhydride group, amine group, carboxyl group, sulfonic acid group, phosphate ester group, and phosphonic acid group, preferably wherein the hydrophilic surface group is directly bound to the backbone of the rigid beads with throughpores or wherein the hydrophilic surface group is bound to the backbone of the rigid beads with throughpores with a bridging (CH₂)₁₋₃ alkyl, as for example in carboxymethyl group, sulfomethyl group or sulfopropyl group.

6. The chromatography method of any one of claims 1 to 5, wherein the target nanoparticles and the hydrophilic mobile phase are fed to the column-packed hydrophilic stationary phase in separate inlets or wherein, prior to step (i), the target nanoparticles are dispersed in the hydrophilic mobile phase, preferably by in-line mixing and/or wherein the hydrophilic mobile phase features a linear flow velocity of at least 10 cm/h, at least 30 cm/h, at least 50 cm/h, at least 100 cm/h, at least 200 cm/h, at least 350 cm/h, at least 500 cm/h, at least 1000 cm/h, at least 1500 cm/h most preferably at least 2000 cm/h.

7. The chromatography method of any one of claims 1 to 6, wherein the target nanoparticles is a mixture of at least two types of target nanoparticles and/or wherein the target nanoparticles, are macromolecules, preferably biological macromolecules including viruses, virus-like particles, viral gene vectors or proteins such as antibodies, preferably wherein the viruses have an empty capsid and/or a capsid that is not empty.

8. The chromatography method of any one of claims 1 to 7, wherein the non-ionic dissolved polymer is a polyol or a poloxamer, preferably wherein the non-ionic dissolved polymer is a polyalkylene glycol, more preferably, a polyethylene glycol (PEG), a polypropylene glycol or mixtures thereof, preferably a PEG, more preferably wherein the PEG is PEG 2000 to PEG 12000, preferably wherein the PEG is PEG 4000 to PEG 10000, more preferably wherein the PEG is PEG 5000 to PEG 8000, most preferably wherein the PEG is PEG 6000, preferably wherein the non-ionic dissolved polymer in the hydrophilic mobile phase is dissolved in a mass fraction of 1% to 40%, preferably 2% to 20%, more preferably 4% to 12%, most preferably 6% to 10%.

9. The chromatography method of any one of claims 1 to 8, wherein the hydrophilic mobile phase, the eluent and/or the optional hydrophilic washing phase comprises water, preferably wherein the hydrophilic mobile phase, the eluent and/or the optional hydrophilic washing phase is an aqueous buffer or water.

10. The chromatography method of any one of claims 1 to 9, wherein the eluent comprises a non-ionic dissolved polymer, preferably wherein the non-ionic dissolved polymer is a non-ionic dissolved polymer according to claims 8, preferably wherein the non-ionic dissolved polymer in the eluent and the non-ionic dissolved polymer in the hydrophilic mobile phase are the same non-ionic dissolved polymer,
preferably wherein the eluent comprises a different mass fraction of the non-ionic dissolved polymer in the eluent compared to the mass fraction of the non-ionic dissolved polymer in the hydrophilic mobile phase, preferably wherein the mass fraction of the non-ionic dissolved polymer in the eluent is smaller and/or wherein the eluent has a different pH, preferably a higher pH, compared to the pH in the hydrophilic mobile phase, and/or wherein the eluent comprises a higher ionic strength, a higher salt concentration, and/or a higher ion-concentration in the eluent compared to the ionic strength, salt concentration and/or ion-concentration of the hydrophilic mobile phase.

11. The chromatography method of any one of claims 1 to 10, wherein the target nanoparticles are detached from the column-packed stationary phase by step elution, by gradient elution or by a mixture thereof, preferably wherein the mass fraction of the non-ionic dissolved polymer in the eluent is decreasing with time and/or wherein the ionic strength and/or ion-concentration and/or salt concentration in the eluent is increasing with time and/or wherein the pH of the eluent is changing with time, preferably wherein the pH of the eluent is increasing with time.

12. **A composition comprising purified and/or separated nanoparticles,** wherein the purified and/or separated nanoparticles are the target nanoparticles obtainable by the chromatography method according to any one of claims 1 to 11.

13. **A composition comprising purified and/or separated nanoparticles for use in medicine, and/or for use in the manufacturing of a medicament or a vaccine,** wherein the composition comprising purified and/or separated nanoparticles is a composition according to claim 12.

14. A method for the depletion of nanoparticles from a liquid target composition, comprising the sequential steps:
(u) dissolving a non-ionic polymer according to claim 8 in the liquid target composition in a concentration according to claim 8;
(v) bringing into contact the liquid target composition containing the non-ionic dissolved polymer with a column-packed hydrophilic stationary phase according to any one of claims 1 to 5, thus allowing the nanoparticles to attach to the column-packed hydrophilic stationary phase and creating a nanoparticle-depleted target composition;
(w) eluting the nanoparticle-depleted target composition,
wherein the nanoparticles are the target nanoparticles according to claim 7.

15. **A nanoparticle-depleted composition,** obtainable by the method of claim 14.
